# NEW EUROPEAN PATENT SPECIFICATION

(11) **EP 1 441 713 B2**
(45) Date of publication and mention of the opposition decision: **14.11.2018**
(45) Mention of the grant of the patent: 08.08.2007
(21) Application number: 02780143.0
(22) Date of filing: 06.11.2002
(51) Int. Cl.: A61K 31/18, A61K 9/20, A61K 9/28, A61K 9/48, A61P 13/08

(54) **MODIFIED RELEASE TAMSULOSIN TABLETS**
TAMSULOSIN TABLETTEN OHNE NAHRUNGSMITTELEFFEKT
COMPRIMES DE TAMSULOSINE A LIBERATION MODIFIEE

(30) Priority: 07.11.2001 US 331055 P
(43) Date of publication of application: 04.08.2004
(60) Divisional application: 05017380.6
(73) Proprietor: Synthon B.V., 6545 CM Nijmegen (NL)
(72) Inventor: LEMMENS, Jacobus, Maria, NL-6585 KM Mook (NL); PLATTEEUW, Johannes, Jan, NL-5215 BL 's-Hertogenbosh (NL); VAN DALEN, Frans, NL-6524 CK Nijmegen (NL); ORTEGA, Arturo, Siles, E-08830 San Boi de Llobregat (ES); ESCOI, Juan, Cucala, E-08830 San Boi de Llobregat (ES)
(74) Representative: Mendivil Gil, Maria Dolores
(86) International application number: PCT/NL2002/000710
(87) International publication number: WO 2003/039531

(56) References cited:
- EP-A- 1 088 551
- WO-A1-03//039530
- US-A- 4 772 475

## Description

### BACKGROUND OF THE INVENTION

The present invention relates to the use of modified release tamsulosin tablets that exhibit little or no food effect and to unit dosage forms made therefrom, in treating or ameliorating the condition of benign prostatic hyperplasia.

Tamsulosin is the common name for 5-[ 2-[[2-(2-ethoxyphenoxy)ethyl]amino] propyl]-2-methoxy-benzenesulfonamide of the formula (1). 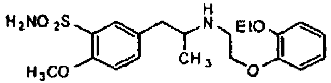 It is disclosed in EP 34432 and US 4731478 as a pharmaceutically active substance having alpha-adrenergic blocking activity that is useful for treatment of cardiac insufficiencies and benign prostatic hyperplasia.

(R)-tamsulosin hydrochloride is marketed under various tradenames, including FLOMAX® (Boehringer Ingelheim) in the U.S., HARNAL® (Yamanouchi) in Japan and OMIVIC® (Yamanouchi) in Europe, for treatment of symptoms of benign prostatic hyperplasia (also known as BPH) such as urinary volume and frequency problems. The approved drug products include a capsule dosage form for oral administration that comprises 0.4 mg of the tamsulosin hydrochloride. The capsule provides controlled release of the tamsulosin and is a once daily dosage form, although two capsules can be used if needed; i.e. a maximum single daily administration of 0.8 mg.

The controlled or modified release commercialized capsule form suffers from a drawback in that it exhibits a food effect. A food effect refers to the difference in bioabsorption or bioavailability of a drug arising from administration to a fasting patient (an empty stomach) versus a fed patient (food in the stomach). For the commercial capsule, the food effect is rather pronounced. For example, it is reported in the labeling information for FLOMAX® that under fasted conditions the Tmax is 4-5 hours, but the Tmax under fed conditions is 6-7 hours. Taking the capsules under fasted conditions results in a 30% increase in bioavailability (AUC) and 40% to 70% increase in peak concentrations (Cmax) compared to fed conditions. Thus, when taken after a meal, the tamsulosin achieves a lower maximum blood plasma concentration; and this peak is achieved later in time. Accordingly, administering after a meal provides a flatter and more controlled release blood plasma profile in comparison to administering under fasting conditions, albeit with a loss in bioavailability.

The commercial capsule labeling instructs the administration to occur in a fed state: after a meal (Japan), after breakfast (Europe) and 30 minutes after the same meal each day (U.S.). It is believed that this dosing is recommended because it provides more consistent results and fewer side effects. Indeed, even though the absorption of the tamsulosin is better under fasted conditions (90+%) then fed conditions, the approved use directs that the tamsulosin capsule be administered under fed conditions.

The commercial capsule form of tamsulosin is believed to correspond to U.S. 4,772,475 (EP 194838, EP 533297). U.S. 4,772,475 discloses controlled-release pharmaceutical dosage forms comprising multiple granulate units containing tamsulosin, microcrystalline cellulose and a release control agent. The granulate gradually releases tamsulosin from the granulate matrix. No discussion occurs surrounding the food effect issue.

Because of the food effect in the commercial capsule tamsulosin product, a patient that takes the capsule while fasting (without a meal) is potentially more likely to experience undesirable side-effects such as dizziness, rhinitis, and/or abnormal ejaculation. It would be beneficial to make a tamsulosin pharmaceutical dosage form that exhibits reduced, little, or even no food effect. In this way, the dosage form would be safer; i.e. even if taken under fasted conditions, the risk of side effects is lessened. Although the food effect of the commercial tamsulosin capsule is well documented, no solution to the food effect problem has been provided thus far.

### SUMMARY OF THE INVENTION

It has now been discovered that a modified release tamsulosin tablet can be formed that has reduced food effect. Accordingly one aspect of the invention relates the use of tamsulosin in the manufacture of a pharmaceutical tablet comprising a tablet matrix having dispersed therein 0.1 to 10 mg of tamsulosin which is (R)-enantiomer of tamsulosin or a pharmaceutically acceptable salt thereof, and optionally having an enteric coating over said matrix, wherein said tablet is a modified release tablet and has a dissolution profile such that in each of the media SIF, FaSSIF, and FeSSIF, said tablet releases not more than 60% of said tamsulosin at 2 hours elapsed time in USP 2 apparatus using 500 ml of said media at 50-100 rpm paddle speed, for treating or ameliorating the condition of benign prostatic hyperplasia, wherein the tablet is taken under fasted conditions.
Preferably the tablet releases at least 20% of the tamsulosin by the 2 hour mark, again in each of the three media. The media serve to model *in vitro* the intestinal conditions encountered *in vivo,* with FaSSIF corresponding to a fasting state and FeSSIF corresponding to a fed state. By having less than 60% of the tamsulosin released at 2 hours under each of the simulated conditions, the tablet demonstrates that neither fed nor fasting conditions are likely to reduce the modified release nature of the drug product.

Another aspect of the invention relates to the of tamsulosin in the manufacture of a monolithic pharmaceutical tablet, comprising 0.1 to 10 mg of tamsulosin which is (R)-enantiomer of tamsulosin or a pharmaceutically acceptable salt thereof, 10 wt% - 90 wt% hydroxypropyl methylcellulose, and a total tablet weight of 10 to 300 mg, for treating or ameliorating the condition of benign prostatic hyperplasia, wherein the tablet is taken under fasted conditions. The tablet exhibits reduced food effect and preferably meets the dissolution profile requirements described above.

In each of these aspects, the tablets can be administered per se, optionally with an enteric coating, but preferably without an enteric coating, or one or more tablets can be encapsulated and administered as one or more capsules. Thus, the invention relates to the use of one or more of the above tablets for the manufacture of a medicament for the treatment or amelioration of the symptoms of benign prostatic hyperplasia, wherein the tablets are taken under fasted conditions.

### BRIEF DESCRIPTION OF THE DRAWINGS

- FIG. 1: is the release curves of tamsulosin capsules manufactured by Yamanouchi Europe in the four described media.
- FIG.2: is the release curves of tamsulosin tablets (batch G) in the four described media.
- FIG.3: is the release curves of tamsulosin tablets (batch H) in the four described media.
- FIG.4: is the release curves of tamsulosin enteric coated tablets (batch G) in the four described media.
- FIG.5: is the release curves of tamsulosin enteric coated tablets (batch H) in the four described media.
- FIG.6: is the release curves of tamsulosin uncoated and coated tablets (batch N) in selected media.

### DETAILED DESCRIPTION OF THE INVENTION

The present invention relates to the use of tamsulosin in the manufacture of modified release tamsulosin tablets for treating or ameliorating the condition of benign prostatic hyperplasia, wherein the tablet is taken under fasted conditions. "Modified release" is used herein in a broad sense and means any tablet that is not an immediate release tablet ; i.e., not a tablet that releases in a dissolution test at least 75% of the tamsulosin within the first 30 minutes in a standard dissolution test (i.e. USP apparatus 2, paddles at 50 rpm, with 500 ml SGF at 3JOC). The tablets exhibit a reduced food effect in comparison to the commercially available tamsulosin capsules. Unlike these capsules, the present invention is based on the discovery that tamsulosin or its pharmaceutically acceptable salt can be formulated into a tablet having controllable food effect properties.

The tamsulosin tablets exhibit a dissolution profile such that in each of SIF, FaSSIF, and FeSSIF,
no more than 60%, preferably 20% to 60%, of the tamsulosin is released at 2 hours. The dissolution test is run with paddles at 50-100 rpm, preferably at 100 rpm, in USP apparatus 2 using 500 ml of the media in which the test is being conducted. In certain embodiments, especially those involving uncoated tablets, the dissolution profile further includes releasing no more than 60%, preferably 20% to 60%, of the tamsulosin in 500 ml of SGF at 2 hours is released in USP apparatus 2, paddles at 50-100 rpm, preferably at 100 rpm. In all dissolution tests for determining the release profile for purposes of the present invention, the media is at a temperature of 37 °C. 500 ml of media is used, assuming one tablet is placed in the apparatus, because it is believed to provide more accurate modeling/prediction of the *in vivo* result.

The dissolution mediums, for purposes of this invention, are defined as follows:
SGF (USP Simulated Gastric Fluid without pepsin) composition:

| | | |
|---|---|---|
| HCl | qs | pH 1.2 |
| NaCl | | 0.2% |
| water | qs | 1000 ml |

SIF (USP Simulated Intestinal Fluid without pancreatin) composition:

| | | |
|---|---|---|
| | KH₂PO₄ | 6.8 g |
| NaOH | qs | pH 6.8 |
| water | qs | 1000 ml |

FeSSIF (Simulated Intestinal Fluid, Fed State) Composition:

| | | |
|---|---|---|
| Acetic acid | | 0.144M |
| NaOH | qs | pH 5 |
| Na Taurocholate | | 15 mM |
| Lecithin | | 4mM |
| KCl | | 0.19 M |
| distilled water | qs | 1000 ml |

pH=5
osmolarity=485-535 mOsm
buffer capacity= 75±2 mEQ/UpH

FaSSIF (Simulated Intestinal Fluid, Fasting State) Composition:

| | | |
|---|---|---|
| KH₂PO₄ | | 0.029 M |
| NaOH | qs | pH 6.8 |
| Na Taurocholate | | 5 mM |
| Lecithin | | 1.5 mM |
| KCI | | 0.22 M |
| distilled water | qs | 1000 ml |

pH=6.8
osmolarity=280-310 mOsm
buffer capacity= 10±2 mEQ/UpH

SGF represents a standard stomach condition. SIF represents a standard intestinal condition. FeSSIF is tailored to better represent the fed state while FaSSIF is tailored to better represent the fasting state. Note that not only are the pH different, but equally important, the osmolarity are also different. FaSSIF and FeSSIF media have been generally used to describe *in vitro in vivo* correlations for immediate release lipophilic, poorly water-soluble drugs (i.e. ketoconazole, danazol, atovaquone, troglitazone, mefenamic acid) but none of the previous studies suggest the application to a modified release low dose composition, and soluble drug such as tamsulosin HCl (volume of any aqueous media needed to dissolve until 10 mg is not more than 500 ml.), neither to a modified-release formulation (including controlled, extended or delayed release).

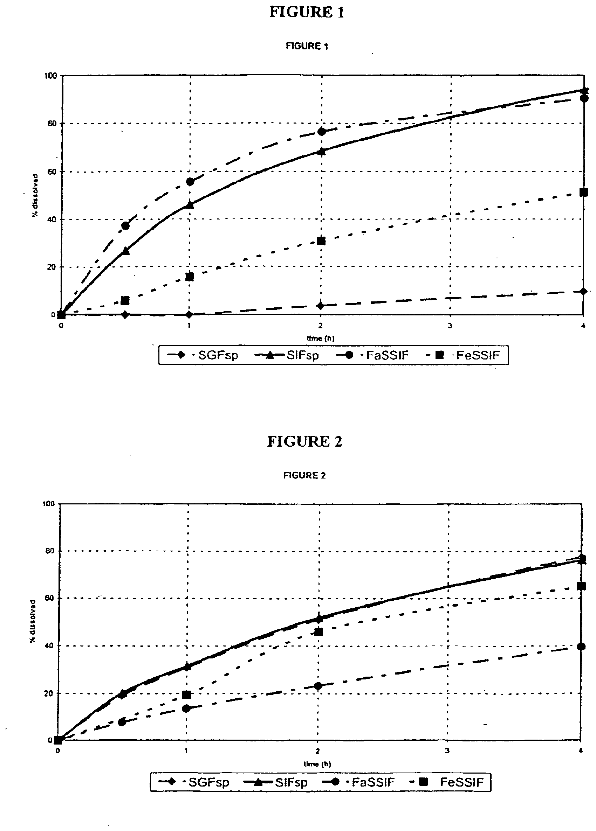

As shown in the subsequent reference example, the commercial capsules manufactured by Yamanouchi Europe, display the following release of tamsulosin at 2 hours elapsed time: more than 60% release in SIF, more than 75% release in FaSSIF while less than 40% is released in FeSSIF and less than 20% is released in SGF (see Figure 1). This divergence of results in the four media, being too fast at pH 6.8 and too slow (delayed release) at pH 1.2, indicates a possible reason for variability in plasma concentration of tamsulosin as indicated before; e.g., because of different gastric emptying and/or changing of gastrointestinal pH. Of course this more rapid release in FaSSIF corresponds well with the *in vivo* observations of a quicker Tmax and a higher Cmax in a fasting state than in a fed state. Accordingly, tablets of the present invention which exhibit not more than 60% tamsulosin release in each of SIF, FaSSIF, and FeSSIF, preferably also in SGF, have a better food effect, i.e., less difference between fed and fasted conditions, than the commercial capsules.

Preferably the tamsulosin tablets release 20% to 60% of the tamsulosin during the first 2 hours of the dissolution test in each of SIF, FaSSIF, and FeSSIF. More preferably, the amount of tamsulosin released at 2 hours in FeSSIF is at least 60% of the amount of tamsulosin released at 2 hours in FaSSIF, under the same paddle speed conditions, preferably at 100 rpm. The tablet is preferably a once daily dose tablet, which exhibits a dissolution profile in SIF within the following ranges:
<40 %in 30 minutes
20-60% in 2 hours
>75% in 6 hours
using USP apparatus 2, using 500 ml of said media with paddles at 100 rpm. More preferably the tablet also exhibits a profile within the above ranges in at least one of, and most preferably both of, FaSSIF and FeSSIF. In some embodiments, the tablet also exhibits a profile within the above ranges in SGF. It should be understood that in meeting the above ranges the dissolution profile in each media need not be identical to each other, although such is contemplated as an embodiment of the invention.

For clarity, the amount or percentage of tamsulosin released at a stated time refers to the cumulative total amount of tamsulosin released from the tablet from the start of the dissolution test up to the stated time. The amount released is determined as the average of the results from six trials; e.g. six tablets, for each media or condition. While apparatus 2 and various conditions have been specified, such is not meant to imply that the same or similar release profiles can not be obtained using different apparatus, such as USP apparatus 1 (basket), or different conditions, such as more or less media, etc. Rather, the above defined apparatus and conditions serve as a convenient way to characterize the inherent properties of the tablets of the invention.

That improved food effect could be obtained is surprising given the performance of the commercial capsule product. In general, the degree of food effect is governed by the type and solubility of the active, the amount/concentration of active, the type and concentration of polymer, and the total mass of the composition. That a modified release low dose tamsulosin tablet having a polymeric matrix could be formulated with reduced food effect, especially without a coating, is unexpected.

The tamsulosin present in the tablet is normally the (R)-enantiomer of tamsulosin within the meaning of tamsulosin or a pharmaceutically acceptable salt thereof. Examples of useful tamsulosin pharmaceutically acceptable salts include tamsulosin hydrochloride, tamsulosin hydrobromide, tamsulosin methane sulfonate, tamsulosin tosylate, tamsulosin besylate, tamsulosin acetate, tamsulosin maleate, tamsulosin tartrate, and tamsulosin citrate. Typically the hydrochloride salt is used.

The amount of (R)-enantiomer of tamsulosin active material present in the tablet is relatively low, generally less than 5%. As used herein all percentages refer to weight percent based on the entire weight of the tablet without taking into account any coating thereon, unless otherwise indicated. Typically the amount of tamsulosin active material is within the range of 0.2 to 1.0 %, preferably 0.2 to 0.8 %, and in many embodiments 0.3 to 0.6 %. In absolute terms, the amount of tamsulosin active material is within the range of 0.1 to 10 mg, generally within the range of 0.1 to 1.2 mg, typically 0.3 to 1.2 mg, and preferably 0.3 to 0.8 mg, expressed in terms of the amount of free base. For example, 0.4 mg of tamsulosin HCl is a preferred amount of tamsulosin which corresponds to 0.367 mg of tamsulosin free base. A preferred embodiment of the present invention contains 0.4 mg +/- 0.04 of (R)-enantiomer of tamsulosin HCl or multiples thereof; i.e., 0.2 or 0.8 mg of (R)-enantiomer of tamsulosin HCI.

The tablets of the present invention further contain a polymeric matrix. Specific examples of suitable polymeric materials include water swellable cellulosic derivatives such as hydroxypropylmethyl cellulose (HPMC), carboxymethyl cellulose, cellulose acetate, hydroxyethyl cellulose, hydroxypropyl cellulose; sodium alginate; acrylates, methacrylates and co-polymers thereof with various co-monomers; and polyvinyl pyrrolidones.

In particular, it has been surprisingly found that while acrylates and methacrylates such as Eudragits® (Rohm) as well as celluloses can provide pH independent release, the celluloses generally provide for better food effect properties. Hydroxyethyl cellulose, hydroxpropyl cellulose, cellulose acetate, sodium alginate, carboxymethyl cellulose and hydroxypropyl methylcellulose (HPMC) are preferred, with HPMC being the most preferred. The amount of HPMC is generally within the range of 10 to 90 %, typically 20 to 60%, preferably 25 to 40 %, more preferably 30 to 40%, still more preferably 30 to 35 %, based on the total weight of the tablet.

The tablets typically contain additional pharmaceutically acceptable excipients, such as diluents, binders, lubricants, glidants, colorants, preservatives, pH-adjusters, etc. The excipients are selected based on the desired physical aspects of the final form, the desired release rate of the active substance from the composition after its ingestion, and the ease/cost of manufacture. In general the tablets of the present invention contain in addition to the polymeric matrix, at least one carbohydrate and/or compressible diluent. Carbohydrates include lactose, mannitol, maltodextrin, cyclodextrins, dextrates, and dextrin. Compressible diluents include any pharmaceutically acceptable diluent that is suitable for direct compression especially calcium phosphates such as calcium hydrogen phosphate dihydrate and anhydrate forms.

In a preferred embodiment of the present invention, the tablet comprises HPMC and a calcium phosphate such as dicalcium phosphate anhydrate. This embodiment preferably additionally contains a carbohydrate such as lactose anhydrate. A lubricant such as magnesium stearate is also preferably included. The relative amounts are not particularly limited, but it is preferred that these two or three excipients (cellulosic polymer, diluents, and lubricant) comprise the majority of the excipients, such as 95% or more. For example, tablets can comprise 25 to 45% HPMC, 0-50% calcium phosphate (or other insoluble diluent), and 0 to 50 % lactose (or other soluble diluent). The following amounts are preferred: 25 to 40% HPMC, 25-40% calcium phosphate, and 25 to 40% lactose. More preferably the tablet contains about 30 to 40% HPMC. Substantially equal amounts of HPMC, calcium phosphate and lactose, i.e. each around 30 to 35% for a total of 90 to 99.9% is a particularly preferred embodiment. Additional excipients, including a lubricant, etc. may also be present. This preferred tablet formulation generally exhibits the preferred dissolution profiles described-above.

The monolithic tablet, is a tablet which does not disintegrate after ingestion to form a plurality of smaller particles from which the active component is finally released. Instead, the product erodes in the body and/or drug diffuses through the polymer gel releasing the active substance. Thus, in the monolithic tablet embodiment, none of the excipients used in the manufacturing process of the invention should serve as a disintegrant.

Any of the tablets may be coated such as with an enteric coat or simply for color or stability reasons. For therapeutic purposes, bioabsorption of tamsulosin in body fluids should preferably proceed in the small intestines. Accordingly, the tablets of the invention may also be protected by a suitable gastro-resistant coating which delays the onset of release of the active component from the tablet matrix during the passage thereof in the stomach, but this is not necessary to obtain the desired profiles. Examples of such suitable material for gastro-resistant coating are cellulose acetate phthalate (CAP) (Aquacoat CPDTM), co-processed polyvinyl acetate phthalate (PVAP) (SureteticTM), cellulose acetate trimellitate (CAT), Eudragit-type polymers (acrylic- methacrylic acid copolymers), hydropropyl methyl cellulose acetate succinate (HPMCAS).

The release property of the coating may be tested also by the same dissolution tests of the uncoated tablets. The preferred properties of a coated tablet are, e.g.:
- Dissolution of the dosage form in SGF, a maximum of 20% of tamsulosin is released in 2 hours.
- In the other media, the coated tablets should comply with the same dissolution profile as specified above.

The tablets-of-the present invention can be used directly as a unit dosage form, with or without coating, or two or more tablets can be combined such as in a capsule to form a unit dose. The unit dosage form contains an effective amount of tamsulosin for treating or ameliorating the disease, symptoms, and/or or conditions associated with BPH, hypertension,, generally from 0.01 to 10.0 mg, preferably 0.1 to 1 mg, in terms of the free base. Preferable are unit doses comprising 0.2, 0.4 or 0.8 mg tamsulosin hydrochloride per se. A unit dose is normally taken from 1 to 3 times daily, preferably once a day as mentioned above. In the case of a capsule, a sufficient number of tablets are provided based on the concentration of the tamsulosin active material therein, so as to provide an effective amount.

Taking the usual therapeutic dose of tamsulosin into consideration, a tablet with a total mass of not more than 400 mg and normally between 10 and 300 mg are preferred. As the therapeutic dose of tamsulosin is relatively low, the overall weight of the tablet is advantageously kept as low as possible. Low overall weight of a tablet increases the relative content of tamsulosin in the tablet and thus improves the content uniformity. Furthermore, a small tablet will have a similar rate of the gastro-intestinal transit as the granulated product; thus, results obtained from in-vitro dissolution tests may better predict the actual bioequivalence with the marketed multiparticulate product. From this aspect, preferred tablet weight within the invention is from 25 to 250 mg, more preferably 40 to 200 mg, though it is not limited to this range. The most preferred tablet weight is within the range of 80 to 100, especially approximately 100 mg.

Accordingly, the tablets of the invention may be either small, whereby- whenever produced in a circular shape- the average diameter thereof is from about 1.5 to about 2.5 mm, or they may be produced as normal tablets, with an average diameter of between 2.5 and 15 mm, more usually 2.5 to 10 mm. Apart from a circular shape, tamsulosin compositions may be compressed into oval, round biconvex, pentagonal circumcircle or other suitable tablet shape.

Tablets of the invention comprising unit dosage amount of tamsulosin may be delivered for immediate use in a suitable package unit comprising advantageously from 5 to 100 tablets. Such package may comprise a blister pack comprising advantageously 10, 14, 20, 28 or 30 tablets, or a plastic or glass container/bottle containing the same amount (5 to 100) of tablets. Any suitable pharmaceutically acceptable package material may be used in production the package unit.

The tablets of the present invention can be made by any suitable process of tabletting. For example, the tablets can be made by wet granulation wherein the granules are first formed and then, optionally with the addition of further excipient(s) compressed into a tablet. Alternatively, the tablets can be made by dry processes such as direct compression or dry granulation, the latter sometimes being referred to as dry compaction. Preferably the tablets are made by a dry technique in view of manufacturing ease and economy. Because of the small amount of tamsulosin present in the tablet, it is preferred that multiple blending and/or milling steps be carried out in any dry process.

Tablets for oral administration of tamsulosin according to the invention may be used in the management of functional treatment of symptomatic benign prostate hypertrophy or hyperplasia (BPH) or other disorders treatable by tamsulosin (the Disorders). Accordingly, the present invention provides for treating or ameliorating the symptoms of benign prostatic hyperplasia which comprises administering to a patient in need thereof under fasted conditions with one or more of any of the above- described tablets. The tablets can be administered in a single capsule.

Tablet compositions of the invention may be also used in medical applications in combination with other agents. The combination may be realized in a form of single combination preparation or by separate administration of drugs containing the above agents.

The invention is further illustrated by the following non-limiting Examples. Reference Example

Commercially available tamsulosin hydrochloride capsules were obtained in Europe and subjected to dissolution testing, the average of six trials, in each of SGF, SIF, FaSSIF, and FeSSIF in 500 ml of each media at paddle speed 100 rpm in USP apparatus 2 at 37 OC. The amount of drug released was determined by an HPLC method using an HPLC Agilent 1100 system. The detection was made with UV at 230 nm. The results, shown in figure 1, indicate that at 2 hours elapsed time: less than 20% tamsulosin is released in SGF, more than 60% tamsulosin is released in SIF, more than 75% tamsulosin is released in FaSSIF, and less than 40% tamsulosin is released in FeSSIF.

### Example 1

Three batches of monolithic tablets were made by progressive mixing and direct compression with the following characteristics:
a) Tablet composition

| | | (%) |
|---|---|---|
| Tamsulosin hydrochloride | 0.4 mg | 0.5 |
| Lactose anhydrous | 26.4 mg | 33.0 |
| Dicalcium phosphate anh. | 26.4 mg | 33.0 |
| Hypromelose (HPMC) | 26.4 mg | 33.0 |
| Magnesium stearate | 0.4 mg | 0.5 |
| Total | 80 mg | 100 |

The difference between batches was only in the viscosity value of hypromelose selected:
Batch A contained METHOCEL K4M CR PREMIUM
Batch B contained METHOCEL K15M CR PREMIUM
Batch C contained METHOCEL K100M CR PREMIUM

b) Modus operandi
Tamsulosin hydrochloride was blended (15 min) with anhydrous lactose in a 1:9 ratio (10% of active substance), milled (15 seconds) and blended again (5 min). This preblend was then mixed with the rest of the lactose, dicalcium phosphate and hypromelose (10 min), and finally magnesium stearate was added and mixed (5 min) to form the precompression blend. This progressive mixing system provided tamsulosin homogeneity in the preblend of 97.2-100.4% and in the precompression blend of 88.1-98.6%). Compression was performed in a Korsch EKO press at standard speed and pressure.
c) Characterization of the produced tablets

| Batch | Weight (mg) | Hardness (N) | Height (mm) | Diameter (mm) | Assay (%) |
|---|---|---|---|---|---|
| A | 82.8 | 52 | 2.63 | 5.98 | 93.6 |
| B | 83.5 | 38 | 2.69 | 5.99 | 88.1 |
| C | 81.8 | 52 | 2.66 | 5.99 | 98.6 |

d) Dissolution studies
Dissolution tests were performed using standard USP apparatus 2, paddles at 50 rpm in 500 ml of SIF. The drug released was determined by an HPLC method using an HPLC Agilent 1100 system. The analysis was performed with a guard column and a C₁₈ analytical column, using an isocratic elution mode, with phosphate buffer pH 6.5 and acetonitrile (65: 35) as eluents. The detection was made with UV at 230 nm.
Results comply with the following specification:
<40 % in 30 minutes
20-60 % in 2 hours
>75 % in 6 hours

### Example 2

Three batches of monolithic tablets were made by progressive mixing and direct compression with the following characteristics:
a) Tablet composition

| | D | E | F |
|---|---|---|---|
| Tamsulosin hydrochloride | 0.4 mg | 0.4 mg | 0.4 mg |
| Lactose anhydrous | 35.2 mg | 30.8 mg | 22.0 mg |
| Dicalcium phosphate anh. | 35.2 mg | 30.8 mg | 22.0 mg |
| Hypromelose (HPMC) | 8.8 mg | 17.6 mg | 35.2 mg |
| Magnesium stearate | 0.4 mg | 0.4 mg | 0.4 mg |
| Total | 80 mg | 80 mg | 80 mg |

The difference between batches was only in the concentration of hypromelose used:
Batch D contained 11% METHOCEL K100M CR PREMIUM
Batch E contained 22% METHOCEL K100M CR PREMIUM
Batch F contained 44% METHOCEL K100M CR PREMIUM

b) Modus operandi
Tamsulosin hydrochloride was blended (15 min) with anhydrous lactose in a 1:9 ratio (10% of active substance), milled (15 seconds) and blended again (5 min). This preblend was then mixed with the rest of the lactose, dicalcium phosphate and hypromelose (10 min), and finally magnesium stearate was added and mixed (5 min). Compression was performed in a Korsch EKO press.
c) Characterization of the produced tablets

| Batch | Weight (mg) | Hardness (N) | Height (mm) | Diameter (mm) | Assay(%) |
|---|---|---|---|---|---|
| D | 80.5 | 19 | 2.65 | 6.0 | 91.9 |
| E | 80.4 | 22 | 2.74 | 6.0 | 94.1 |
| F | 80.3 | 22 | 2.97 | 6.0 | 90.5 |

d) Dissolution studies
Dissolution tests were performed using USP apparatus 1, baskets at 100 rpm and USP apparatus 2, paddles, at 50 rpm, both in 500 ml of SIF. The drug released was determined by an HPLC method using an HPLC Agilent 1100 system. The analysis was performed with a guard column and a C₁₈ analytical column, using an isocratic elution mode, with phosphate buffer pH 6.5 and acetonitrile (65:35) as eluents. The detection was made with UV at 230 nm.
Results vary according to HPMC concentration, and in each condition, there is a wide range of curves complying with the intended specification. These results can be extrapolated to other bio-relevant dissolution conditions.

### Example 3

Two batches of monolithic tablets were made by progressive mixing and direct compression with the following characteristics:
a) Tablet composition

| | | (%) |
|---|---|---|
| Tamsulosin hydrochloride | 0.4 mg | 0.5 |
| Lactose anhydrous | 25.6 mg | 32.0 |
| Dicalcium phosphate anh. | 25.6 mg | 32.0 |
| Hypromelose (HPMC) | 28.0 mg | 35.0 |
| Magnesium stearate | 0.4 mg | 0.5 |
| Total | 80 mg | 100 |

The differences between both batches were mainly scaling-up, mixing times and physical parameters.
Batch G scaled-up to 20000 units
Batch H scaled-up to 40000 units

b) Modus operandi
Tamsulosin hydrochloride was blended (Turbula; 15 min) with anhydrous lactose in a 1:9 ratio (10% of active substance), milled (IKA; 30 seconds) and blended again (Turbula; 5 min). This pre-blend was then mixed with the rest of the lactose, dicalcium phosphate and hypromelose (Bohle LM40). Three progressive mixing times (15, 30 and 45 minutes) were evaluated for batch D and homogeneity was excellent in all cases (tamsulosin assay of 101.2 %, 101.7% and 102.1 %). Batch E was mixed for only 10 minutes and acceptable homogeneity was also reached. Sieving of dry blends and excipients was done as required to obtain homogeneity. Finally magnesium stearate was sieved, added and mixed (Bohle LM40; 5 min). The precompression blends were compressed in either an eccentric press Korsch EKO or in a rotary press Korsch XL100 (about 15000-30000 tablets). Compression performed in Korsch XL100 instrumented rotary press was done at high speeds with standard precompression and pressure. Tablet hardness in both batches was changed to study dissolution performance.
c) Characterization of the produced tablets

| Batch | Weight (mg) | Hardness (N) | Height (mm) | Diameter (mm) | Assay(%) |
|---|---|---|---|---|---|
| G | 80.9 | 85 | 2.55 | 6.00 | 103.2 |
| H | 76.4 | 39 | 2.38 | 5.97 | 98.2 |

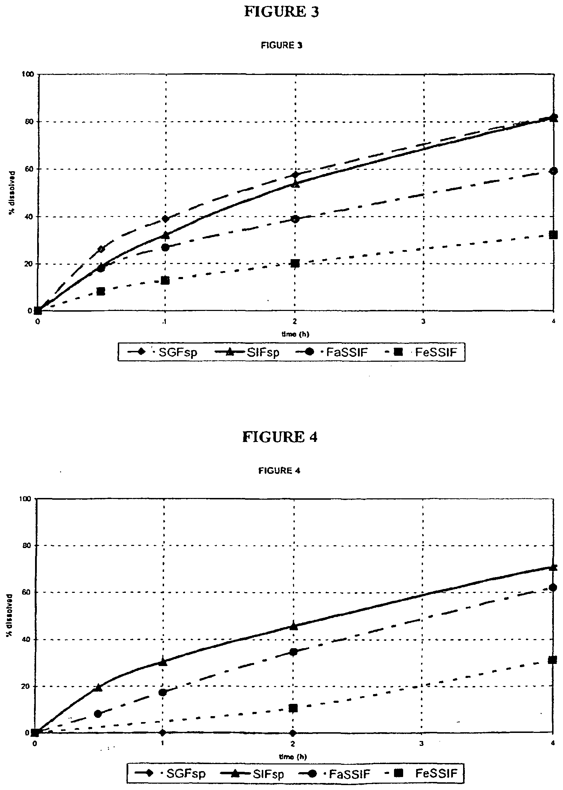

d) Dissolution studies
Dissolution tests were performed using standard USP apparatus 2, paddles at 100 rpm in 500 ml of SGF, SIF, FaSSIF and FeSSIF. The drug released was determined by an HPLC method using an HPLC Agilent 1100 system. The analysis was performed with a guard column and a C₁₈ analytical column, using an isocratic elution mode, with phosphate buffer pH 6.5 and acetonitrile (65:35) as eluents. The detection was made with UV at 230 nm. The corresponding curves are given in Figures 2(G) and 3 (H). Results in SGF and SIF comply with the following specification:
<40 %in 30 minutes
20-60% in 2 hours
>75% in 6 hours

e) coating
These tablets were then coated with an enteric polymer (polymethacrylate type C) based on Eudragit L30D55, with additives including triethylcitrate and talc, or with Acryi-Eze ® (available from Colorcon).
f) Dissolution studies
Dissolution tests of the coated batches were performed using standard USP apparatus 2, paddles at 100 rpm in 500 ml of SGF, SIF, FaSSIF and FeSSIF. The drug released was determined by an HPLC method using an HPLC Agilent 1100 system. The analysis was performed with a guard column and a C₁₈ analytical column, using an isocratic elution mode, with phosphate buffer pH 6.5 and acetonitrile (65:35) as eluents. The detection was made with UV at 230 nm. The corresponding curves are given in Figures 4 (coated G) and 5 (coated H).
Results in SIF comply with the following specification:
<40 %in 30 minutes
20-60% in 2 hours
>75% in 6 hours

### Example 4

Two batches of tablets were manufactured by a process that includes dry compaction, milling, mixing and compression.
a) Tablet composition

| | I | J |
|---|---|---|
| Tamsulosin hydrochloride | 0.4 mg | 0.4 mg |
| Lactose | 65.6 mg | 215.6 mg |
| Hypromelose (HPMC) | 33.0 mg | 33.0 mg |
| Magnesium stearate | 1.0 mg | 1.0 mg |
| Total | 100 mg | 250 mg |

Batch I: Monolithic tablet, 6 mm diameter. Contains 33.0% of HPMC K15M P
Batch J: Monolithic tablet, 9 mm diameter. Contains 13.2% of HPMC K15M P

b) Modus operandi
Tamsulosin was blended (15 min), milled (15 seconds) and blended again (5 min), with anhydrous lactose in a 1:9 ratio (10% of active substance). This preblend was then mixed with the rest of lactose, hypromelose and 25% of magnesium stearate (10 min), compacted in Chilsonator (Fitz-Patrick) and milled in Fitz-Mill (Fitz-Patrick), finally the rest of magnesium stearate (75%) was added and then mixed (15 min). Compression was performed in a Korsch EKO press at standard speed and pressure.
c) Characterization of the produced tablets.

| Batch | Weight (mg) | Hardness (N) | Height (mm) | Diameter (mm) | Lubrication coefficient |
|---|---|---|---|---|---|
| I | 103.3 | 31 | 3.42 | 6.0 | 98.4 |
| J | 251.6 | 42 | 3.71 | 9.0 | 100 |

### Example 5

Two batches of tablets were manufactured with different active concentration, tablet geometries and total mass.
a) Tablet composition

| | K | L | M |
|---|---|---|---|
| Tamsulosin hydrochloride | 0.4 mg | 0.2 mg | 0.2 mg |
| Lactose anhydrous | 25.6 mg | 12.9 mg | 129.0 mg |
| Dicalcium phosphate anh. | 25.6 mg | 12.7 mg | 128.8 mg |
| Hypromelose (HPMC) | 28.0 mg | 14.0 mg | 140.0 mg |
| Magnesium stearate | 0.4 mg | 0.2 mg | 2.0 mg |
| Total | 80 mg | 40 mg | 400 mg |

b) Modus operandi
Micronised tamsulosin was blended progressively with anhydrous lactose. This preblend was then mixed with the rest of lactose and hypromelose and after with magnesium stearate to provide in all cases enough homogeneity. Compression was performed in a Korsch EKO press at standard speed and pressures.
c) Characterization of the produced tablets

| Batch | Weight (mg) | Hardness (N) | Height (mm) | Diameter (mm) | Assay (%) |
|---|---|---|---|---|---|
| K (special shape) | 84.1 | 53 | 2.65 | n.a. | 90.5 |
| L | 40.6 | 36 | 2.01 | 5.00 | 94.1 |
| M | 398.1 | 132 | 4.57 | 10.05 | 90.5 |

### Example 6

A batch of monolithic tablets was manufactured to check dissolution specifications.
a) Tablet composition

| | | (%) |
|---|---|---|
| Tamsulosin hydrochloride | 0.4 mg | 0.5 |
| Lactose anhydrous | 31.6 mg | 39.5 |
| Dicalcium phosphate anh. | 31.6 mg | 39.5 |
| Hypromelose (HPMC) | 16.0 mg | 20.0 |
| Magnesium stearate | 0.4 mg | 0.5 |
| Total | 80 mg | 100 |

Batch N scale-up was performed using micronised drug substance and less mixing steps. 40000 units were manufactured.
b) Modus operandi
Micronised tamsulosin hydrochloride was blended (Bohle LM40; 15 min) with hypromelose. This pre-blend was then mixed with lactose and dicalcium phosphate (Bohle LM40; 15 minutes). Sieving of dry blends and excipients was done as required to obtain homogeneity. Finally magnesium stearate was sieved, added and mixed, two times (Bohle LM40; 5 and 15 minutes).
The precompression blends were then compressed. Compression was performed in a Korsch XL 100 instrumented rotary press was done at high speeds with standard precompression and pressure. Characterisation of the tablets is presented below:
c) Characterization of the produced tablets

| Batch | Weight (mg) | Hardness (N) | Height (mm) | Diameter (mm) | Assay(%) |
|---|---|---|---|---|---|
| N | 80.7 | 47 | 2.50 | 6.05 | 104.0 |

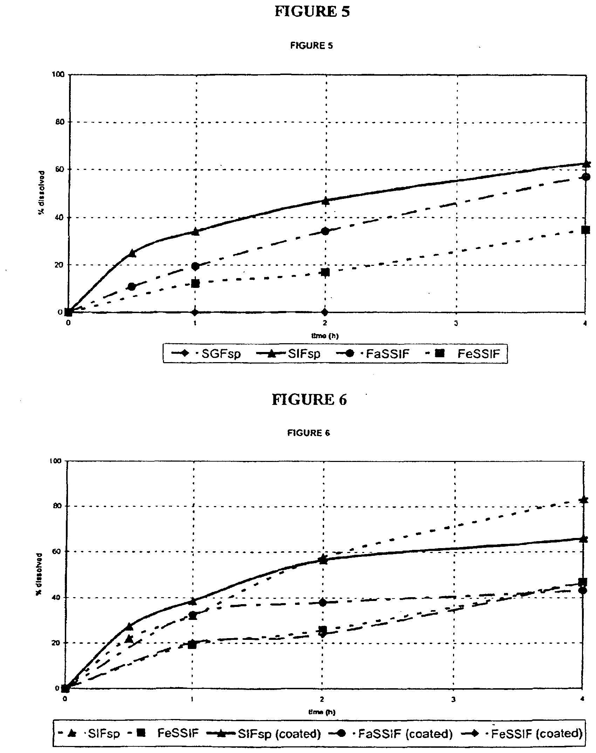

d) coating
This tablets were then coated with an enteric polymer (Acryi-Eze ®available from Colorcon).
e) Dissolution studies
Dissolution tests of the coated batches were performed using standard USP apparatus 2, paddles, at 100 rpm in 500 ml of SGF, SIF, FaSSIF and FeSSIF. The drug released was determined by an HPLC method using an HPLC Agilent 1100 system. The analysis was performed with a guard column and a C₁₈ analytical column, using an isocratic elution mode, with phosphate buffer pH 6.5 and acetonitrile (65:35) as eluents. The detection was made with UV at 230nm. The corresponding curves are given in Figure 6.
Results in all media comply with the following specification:
<40 %in 30 minutes
20-60% in 2 hours
>75% in 6 hours

## Claims

1. Use of tamsulosin in the manufacture of a pharmaceutical tablet comprising a tablet matrix having dispersed therein 0.1 to 10 mg of tamsulosin which is (R)-enantiomer of tamsulosin or a pharmaceutically acceptable salt thereof, and optionally having an enteric coating over said matrix, wherein said tablet is a modified release tablet and has a dissolution profile such that in each of the media SIF, FaSSIF, and FeSSIF, said tablet releases not more than 60% of said tamsulosin at 2 hours elapsed time in USP 2 apparatus using 500 ml of said media at 50-100 rpm paddle speed, for treating or ameliorating the condition of benign prostatic hyperplasia, wherein the tablet is taken under fasted conditions.

2. Use of tamsulosin according to claim 1, wherein said dissolution profile is measured using 100 rpm paddle speed.

3. Use of tamsulosin according to claim 1 or 2, wherein said dissolution profile exhibits a release of at least 20% in each of said media at 2 hours elapsed time.

4. Use of tamsulosin according to claim 1-3, wherein said tablet has a dissolution profile wherein the amount of tamsulosin released at 2 hours in FeSSIF media is at least 50% the amount released at 2 hours in FaSSIF media.

5. Use of tamsulosin according to claim 4, wherein the amount of tamsulosin released at 2 hours in FeSSIF media is at least 60% the amount released at 2 hours in FaSSIF media.

6. Use of tamsulosin according to claim 1-5, wherein said tablet has a dissolution profile wherein said tablet releases not more than 60% of said tamsulosin at 2 hours elapsed time in USP 2 apparatus using 500 ml of SGF media at 100 rpm paddle speed.

7. Use of tamsulosin according to claim 1-6, wherein said dissolution profile includes releasing less than 40% of the tamsulosin in 30 minutes, 20 - 60% of the tamsulosin in 2 hours, and greater than 75% of the tamsulosin in 6 hours in USP 2 apparatus using 500 ml of SIF media at 100 rpm paddle speed.

8. Use of tamsulosin according to claim 1-7, wherein said dissolution profile includes relasing less than 40% of the tamsulosin in 30 minutes, 20 - 60% of the tamsulosin in 2 hours, and greater than 75% of the tamsulosin in 6 hours in USP 2 apparatus using 500 ml of SGF media at 100 rpm paddle speed.

9. Use of tamsulosin according to claim 1-8, wherein said tablet has an enteric coating.

10. Use of tamsulosin according to claims 1-9, wherein the material of the coating comprises at least one compound selected from the group consisting of cellulose acetate phthalate (CAP) (Aquacoat CPD™), co-processed polyvinyl acetate phthalate (PVAP) (Sureteric™), cellulose acetate trimellitate (CAT), Eudragit-type polymers (acrylic-methacrylic acid copolymers), hydroxypropyl methyl cellulose acetate succinate (HPMCAS).

11. Use of tamsulosin according to claim 1-8, wherein said tablet does not have an enteric coating.

12. Use of tamsulosin according to claim 11, wherein said tablet is uncoated.

13. Use of tamsulosin according to claim 1-12, wherein said tablet matrix comprises a polymeric matrix.

14. Use of tamsulosin according to claim 1-13, wherein the matrix component is selected from the group comprising a water swellable cellulosic derivative; sodium alginate; acrylates, metacrylates and co-polymers thereof with various co-monomers; and polyvinyl pyrrolidones.

15. Use of tamsulosin according to claim 14, wherein the water swellable cellulosic derivative is hydroxypropylmethyl cellulose (HMPC), carboxymethyl cellulose, cellulose acetate, hydroxyethyl cellulose, hydroxypropyl cellulose.

16. Use of tamsulosin according to claim 15, wherein said tablet matrix comprises hydroxypropyl methylcellulose.

17. Use of tamsulosin according to claim 15-16, wherein said tablet comprises said hydroxypropyl methylcellulose in an amount within the range of 10 wt% - 90 wt%.

18. Use of tamsulosin according to claim 17, wherein said tablet comprises said hydroxypropyl methylcellulose in an amount within the range of 25 wt% - 40 wt%.

19. Use of tamsulosin according to claim 18, wherein said tablet comprises said hydrxoypropyl methylcellulose in an amount within the range of 30 wt% - 35 wt%.

20. Use of tamsulosin according to claim 1-19, wherein said (R)-enantiomer of tamsulosin or pharmaceutically acceptable salt thereof is contained in an amount of 0.2 to 1.0 %.

21. Use of tamsulosin according to claim 20, wherein said (R)-enantiomer of tamsulosin or pharmaceutically acceptable salt thereof is contained in an amount of 0.2 to 0.8 %.

22. Use of tamsulosin according to claim 1-21, wherein said (R)-enantiomer of tamsulosin of pharmaceutically acceptable salt thereof is tamsulosin hydrochloride and said tamsulosin hydrochloride is contained in an amount of 0.4mg +/- 0.04.

23. Use of tamsulosin according to claim 1-22, which further comprises at least one pharmaceutically acceptable excipient selected from the group consisting of a carbohydrate and a compressible diluent.

24. Use of tamsulosin according to claim 23, which further comprises lactose.

25. Use of tamsulosin according to claim 23-24, which comprises a calcium phosphate.

26. Use of tamsulosin according to claim 1-25, which comprises lactose, HPMC, a calcium phosphate, and magnesium stearate.

27. Use of tamsulosin according to claim 1-26, wherein said tablet is a once daily dose tablet.

28. Use of tamsulosin according to the claims 1-27, being a monolithic tablet, which does not comprise a disintegrant.

29. Use of tamsulosin in the manufacture of a monolithic pharmaceutical tablet, comprising 0.1 to 10 mg of tamsulosin which is (R)-enantiomer of tamsulosin or a pharmaceutically acceptable salt thereof, 10 wt% - 90 wt% hydroxypropyl methylcellulose, and a total tablet weight of 10 to 300 mg, for treating or ameliorating the condition of benign prostatic hyperplasia, wherein the tablet is taken under fasted conditions.

30. Use of tamsulosin according to claim 29, wherein said total tablet weight is within the range of 25 to 250 mg.

31. Use of tamsulosin according to claim 30, wherein said total tablet weight is within the range of 80 to 100 mg.

32. Use of tamsulosin according to claims 29-31, which comprises said hydroxypropyl methylcellulose in an amount within the range of 25 wt% - 40 wt%.

33. Use of tamsulosin according to claims 29-32, which comprises said hydroxypropyl methylcellulose in an amount within the range of 30 wt% - 40 wt%.

34. Use of tamsulosin according to claims 29-33, wherein said tablet comprises said hydroxypropyl methylcellulose in an amount within the range of 30 wt% - 35 wt%.

35. Use of tamsulosin according to claims 29-34, wherein said tablet further comprises a calcium phosphate, lactose, mannitol, or a combination thereof.

36. Use of tamsulosin according to claim 35, wherein said tablet comprises dibasic calcium phosphate anhydrous.

37. Use of tamsulosin according to claim 29-36, which does not contain an enteric coating.

## Patentansprüche

1. Verwendung von Tamsulosin in der Herstellung einer pharmazeutischen Tablette, umfassend eine Tablettenmatrix, die darin dispergiert 0,1 bis 10 mg Tamsulosin aufweist, das das (R)-Enantiomer von Tamsulosin oder ein pharmazeutisch verträgliches Salz davon ist, und gegebenenfalls mit einer magensaftresistenten Beschichtung über der Matrix, wobei die Tablette eine Tablette mit modifizierter Freisetzung ist und ein solches Lösungsprofil aufweist, dass in jedem der Medien SIF, FaSSIF und FeSSIF die Tablette nicht mehr als 60% des Tamsulosins bei 2 Stunden Zeitablauf im USP 2-Apparat unter Verwendung von 500 ml der Medien bei 50-100 UpM Paddelgeschwindigkeit freisetzt, zum Behandeln oder Lindern des Zustands von benigner Prostatahyperplasie, wobei die Tablette unter Nüchternbedingungen eingenommen wird.

2. Verwendung von Tamsulosin nach Anspruch 1, wobei das Lösungsprofil unter Verwendung von 100 UpM Paddelgeschwindigkeit gemessen wird.

3. Verwendung von Tamsulosin nach Anspruch 1 oder 2, wobei das Lösungsprofil eine Freisetzung von mindestens 20% in jedem der Medien bei 2 Stunden Zeitablauf aufweist.

4. Verwendung von Tamsulosin nach Anspruch 1-3, wobei die Tablette ein Lösungsprofil aufweist, wobei die Menge an Tamsulosin, die bei 2 Stunden in FeSSIF-Medien freigesetzt wird, mindestens 50% der Menge beträgt, die bei 2 Stunden in FaSSIF-Medien freigesetzt wird.

5. Verwendung von Tamsulosin nach Anspruch 4, wobei die Menge an Tamsulosin, die bei 2 Stunden in FeSSIF-Medien freigesetzt wird, mindestens 60% der Menge beträgt, die bei 2 Stunden in FaSSIF-Medien freigesetzt wird.

6. Verwendung von Tamsulosin nach Anspruch 1-5, wobei die Tablette ein Lösungsprofil aufweist, wobei die Tablette nicht mehr als 60% des Tamsulosins bei 2 Stunden Zeitablauf im USP 2-Apparat unter Verwendung von 500 ml SGF-Medien bei 100 UpM Paddelgeschwindigkeit freisetzt.

7. Verwendung von Tamsulosin nach Anspruch 1-6, wobei das Lösungsprofil Freisetzen von weniger als 40% des Tamsulosins in 30 Minuten, 20-60% des Tamsulosins in 2 Stunden, und größer als 75% des Tamsulosins in 6 Stunden im USP 2-Apparat unter Verwendung von 500 ml SIF-Medien bei 100 UpM Paddelgeschwindigkeit einschließt.

8. Verwendung von Tamsulosin nach Anspruch 1-7, wobei das Lösungsprofil Freisetzen von weniger als 40% des Tamsulosins in 30 Minuten, 20-60% des Tamsulosins in 2 Stunden, und größer als 75% des Tamsulosins in 6 Stunden im USP 2-Apparat unter Verwendung von 500 ml SGF-Medien bei 100 UpM Paddelgeschwindigkeit einschließt.

9. Verwendung von Tamsulosin nach Anspruch 1-8, wobei die Tablette eine magensaftresistente Beschichtung aufweist.

10. Verwendung von Tamsulosin nach Ansprüchen 1-9, wobei das Material der Beschichtung mindestens eine Verbindung umfasst, die aus der Gruppe bestehend aus Celluloseacetatphthalat (CAP) (Aquacoat CPD™), coprozessiertem Polyvinylacetatphthalat (PVAP) (Sureteric™), Celluloseacetattrimellitat (CAT), Polymeren vom Eudragit-Typ (Acryl-Methacrylsäure-Copolymere), Hydroxypropylmethylcellulose-Acetatsuccinat (HPMCAS) ausgewählt ist.

11. Verwendung von Tamsulosin nach Anspruch 1-8, wobei die Tablette keine magensaftresistente Beschichtung aufweist.

12. Verwendung von Tamsulosin nach Anspruch 11, wobei die Tablette unbeschichtet ist.

13. Verwendung von Tamsulosin nach Anspruch 1-12, wobei die Tablettenmatrix eine Polymermatrix umfasst.

14. Verwendung von Tamsulosin nach Anspruch 1-13, wobei die Matrixkomponente aus der Gruppe umfassend ein wasserquellbares Cellulosederivat; Natriumalginat; Acrylate; Methacrylate und Copolymere davon mit verschiedenen Comonomeren; und Polyvinylpyrrolidone ausgewählt ist.

15. Verwendung von Tamsulosin nach Anspruch 14, wobei das wasserquellbare Cellulosederivat Hydroxypropylmethylcellulose (HMPC), Carboxymethylcellulose, Celluloseacetat, Hydroxyethylcellulose, Hydroxypropylcellulose ist.

16. Verwendung von Tamsulosin nach Anspruch 15, wobei die Tablettenmatrix Hydroxypropylmethylcellulose umfasst.

17. Verwendung von Tamsulosin nach Anspruch 15-16, wobei die Tablette die Hydroxypropylmethylcellulose in einer Menge umfasst, die im Bereich von 10 Gew.-% - 90 Gew.-% liegt.

18. Verwendung von Tamsulosin nach Anspruch 17, wobei die Tablette die Hydroxypropylmethylcellulose in einer Menge umfasst, die im Bereich von 25 Gew.-% - 40 Gew.-% liegt.

19. Verwendung von Tamsulosin nach Anspruch 18, wobei die Tablette die Hydroxypropylmethylcellulose in einer Menge umfasst, die im Bereich von 30 Gew.-% - 35 Gew.-% liegt.

20. Verwendung von Tamsulosin nach Anspruch 1-19, wobei das (R)-Enantiomer von Tamsulosin oder das pharmazeutisch verträgliche Salz davon in einer Menge von 0,2 bis 1,0 % enthalten ist.

21. Verwendung von Tamsulosin nach Anspruch 20, wobei das (R)-Enantiomer von Tamsulosin oder das pharmazeutisch verträgliche Salz davon in einer Menge von 0,2 bis 0,8 % enthalten ist.

22. Verwendung von Tamsulosin nach Anspruch 1-21, wobei das (R)-Enantiomer von Tamsulosin oder das pharmazeutisch verträgliche Salz davon Tamsulosin-Hydrochlorid ist, und das Tamsulosin-Hydrochlorid in einer Menge von 0,4 mg +/- 0,04 enthalten ist.

23. Verwendung von Tamsulosin nach Anspruch 1-22, das weiterhin mindestens einen pharmazeutisch verträglichen Hilfsstoff umfasst, der aus der Gruppe bestehend aus einem Kohlenhydrat und einem komprimierbaren Verdünnungsmittel ausgewählt ist.

24. Verwendung von Tamsulosin nach Anspruch 23, das weiterhin Lactose umfasst.

25. Verwendung von Tamsulosin nach Anspruch 23-24, das ein Calciumphosphat umfasst.

26. Verwendung von Tamsulosin nach Anspruch 1-25, das Lactose, HPMC, ein Calciumphosphat und Magnesiumstearat umfasst.

27. Verwendung von Tamsulosin nach Anspruch 1-26, wobei die Tablette eine einmal tägliche Dosierungstablette ist.

28. Verwendung von Tamsulosin nach den Ansprüchen 1-27, die eine monolithische Tablette ist, die kein Sprengmittel umfasst.

29. Verwendung von Tamsulosin in der Herstellung einer monolithischen pharmazeutischen Tablette, die 0,1 bis 10 mg Tamsulosin, das das (R)-Enantiomer von Tamsulosin oder ein pharmazeutisch verträgliches Salz davon ist, 10 Gew.-% - 90 Gew.-% Hydroxypropylmethylcellulose, und ein Gesamttablettengewicht von 10 bis 300 mg umfasst, zum Behandeln oder Lindern des Zustands von benigner Prostatahyperplasie, wobei die Tablette unter Nüchternbedingungen eingenommen wird.

30. Verwendung von Tamsulosin nach Anspruch 29, wobei das Gesamttablettengewicht im Bereich von 25 bis 250 mg liegt.

31. Verwendung von Tamsulosin nach Anspruch 30, wobei das Gesamttablettengewicht im Bereich von 80 bis 100 mg liegt.

32. Verwendung von Tamsulosin nach Ansprüchen 29-31, die die Hydroxypropylmethylcellulose in einer Menge innerhalb des Bereichs von 25 Gew.-% - 40 Gew.-% umfasst.

33. Verwendung von Tamsulosin nach Ansprüchen 29-32, die die Hydroxypropylmethylcellulose in einer Menge innerhalb des Bereichs von 30 Gew.-% - 40 Gew.-% umfasst.

34. Verwendung von Tamsulosin nach Ansprüchen 29-33, wobei die Tablette die Hydroxypropylmethylcellulose in einer Menge innerhalb des Bereichs von 30 Gew.-% - 35 Gew.-% umfasst.

35. Verwendung von Tamsulosin nach Ansprüchen 29-34, wobei die Tablette weiterhin ein Calciumphosphat, Lactose, Mannitol oder eine Kombination davon umfasst.

36. Verwendung von Tamsulosin nach Anspruch 35, wobei die Tablette wasserfreies dibasisches Calciumphosphat umfasst.

37. Verwendung von Tamsulosin nach Anspruch 29-36, die keine magensaftresistente Beschichtung enthält.

## Revendications

1. Utilisation de tamsulosine pour la fabrication d'un comprimé pharmaceutique comportant une matrice de comprimé dans laquelle sont dispersés 0,1 à 10 mg de tamsulosine, à savoir un énantiomère (R) de tamsulosine, ou d'un sel pharmaceutiquement acceptable en dérivant et comportant éventuellement un enrobage entérique recouvrant ladite matrice, ledit comprimé étant un comprimé à libération modifiée et présentant un profil de dissolution tel que dans chacun des milieux SIF, FaSSIF et FeSSIF ledit comprimé ne libère pas plus de 60% de ladite tamsulosine au bout de 2 heures écoulées dans l'appareil USP 2 utilisant 500 ml dudit milieu sous une vitesse de pales de 50-100 t/min, pour le traitement ou l'amélioration de l'hyperplasie bénigne de la prostate, ledit comprimé étant pris à jeun.

2. Utilisation de tamsulosine selon la revendication 1, dans laquelle ledit profil de dissolution est mesuré sous une vitesse de pale de 100 t/min.

3. Utilisation de tamsulosine selon la revendication 1 ou 2, dans laquelle ledit profil de dissolution présente une libération d'au moins 20% dans chacun desdits milieux au bout de 2 heures de maintien.

4. Utilisation de tamsulosine selon les revendications 1-3, dans laquelle ledit comprimé présente un profil de dissolution dans lequel la quantité de tamsulosine libérée au bout de 2 heures de maintien dans les milieux FeSSIF est au moins égale à 50% de la quantité libérée au bout de 2 heures de maintien dans les milieux FaSSIF.

5. Utilisation de tamsulosine selon la revendication 4, dans laquelle la quantité de tamsulosine libérée au bout de 2 heures dans les milieux FeSSIF est d'au moins 60% de la quantité libérée au bout de 2 heures de maintien dans les milieux FaSSIF.

6. Utilisation de tamsulosine selon les revendications 1-5, dans laquelle ledit comprimé présente un profil de dissolution dans lequel ledit comprimé ne libère pas plus de 60% de ladite tamsulosine au bout de 2 heures écoulées dans l'appareil USP 2 utilisant 500 ml de milieu SGF sous une vitesse de pales de 100 t/min.

7. Utilisation de tamsulosine selon les revendications 1-6, dans laquelle ledit profil de dissolution comprend la libération de moins de 40% de la tamsulosine en 30 minutes, 20-60% de la tamsulosine en 2 heures et de plus de 75% de la tamsulosine en 6 heures dans l'appareil USP 2 utilisant 500 ml de milieu SIF à une vitesse de pale de 100 t/min.

8. Utilisation de tamsulosine selon les revendications 1-7, dans laquelle ledit profil de dissolution comprend la libération de moins de 40% de la tamsulosine en 30 minutes, 20-60% de la tamsulosine en 2 heures et de plus de 75% de la tamsulosine en 6 heures dans l'appareil USP 2 en utilisant 500 ml de milieu SGF à une vitesse de pale de 100 t/min.

9. Utilisation de tamsulosine selon les revendications 1-8, dans laquelle ledit comprimé comporte un enrobage entérique.

10. Utilisation de tamsulosine selon les revendications 1-9, dans laquelle le matériau du revêtement comprend au moins un composé choisi dans le groupe constitué par le phtalate d'acétate de cellulose (CAP) (Aquacoat CPD™), le phtalate d'acétate de polyvinyle co-traité (PVAP) (Sureteric™), le trimellitate d'acétate de cellulose (CAT), les polymères de type Eudragit (copolymères d'acide acrylique et méthacrylique), le succinate d'acétate d'hydroxypropylméthylcellulose (HPMCAS).

11. Utilisation de tamsulosine selon les revendications 1-8, dans laquelle ledit comprimé ne comporte pas de revêtement entérique.

12. Utilisation de tamsulosine selon la revendication 11, dans laquelle ledit comprimé est non enrobé.

13. Utilisation de tamsulosine selon les revendications 1-12, dans laquelle ladite matrice de comprimé comprend une matrice polymèrique.

14. Utilisation de tamsulosine selon les revendications 1-13, dans laquelle le composant matriciel est choisi dans le groupe comprenant un dérivé cellulosique gonflable dans l'eau, l'alginate de sodium, les acrylates, les méthacrylates et leurs copolymères avec divers co-monomères, et les polyvinylpyrrolidones.

15. Utilisation de tamsulosine selon la revendication 14, dans laquelle le dérivé cellulosique gonflable dans l'eau est l'hydroxypropylméthylcellulose (HMPC), la carboxyméthylcellulose, l'acétate de cellulose, l'hydroxyéthylcellulose, l'hydroxypropylcellulose.

16. Utilisation de tamsulosine selon la revendication 15, dans laquelle ladite matrice de comprimé comprend de l'hydroxypropylméthylcellulose.

17. Utilisation de tamsulosine selon les revendications 15 à 16, dans laquelle ledit comprimé comprend ladite hydroxypropylméthylcellulose en une quantité dans la gamme de 10% en poids à 90% en poids.

18. Utilisation de tamsulosine selon la revendication 17, dans laquelle ledit comprimé comprend ladite hydroxypropylméthylcellulose en une quantité dans la gamme de 25% en poids à 40% en poids.

19. Utilisation de tamsulosine selon la revendication 18, dans laquelle ledit comprimé comprend ladite hydroxypropylméthylcellulose en une quantité dans la gamme de 30% en poids à 35% en poids.

20. Utilisation de tamsulosine selon les revendications 1-19, dans laquelle ledit énantiomère (R) de la tamsulosine ou un de ses sels pharmaceutiquement acceptables, est contenu en une quantité de 0,2 à 1,0%.

21. Utilisation de tamsulosine selon la revendication 20, dans laquelle ledit énantiomère (R) de la tamsulosine ou un de ses sels pharmaceutiquement acceptables est contenu en une quantité de 0,2 à 0,8%.

22. Utilisation de tamsulosine selon les revendications 1-21, dans laquelle ledit énantiomère (R) de la tamsulosine ou un de ses sels pharmaceutiquement acceptables est le chlorhydrate de tamsulosine et ledit chlorhydrate de tamsulosine est contenu en une quantité de 0,4 mg ± 0,04.

23. Utilisation de tamsulosine selon les revendications 1-22, qui comprend en outre au moins un excipient pharmaceutiquement acceptable choisi dans le groupe constitué d'un hydrate de carbone et d'un diluant compressible.

24. Utilisation de tamsulosine selon la revendication 23, qui comprend en outre du lactose.

25. Utilisation de tamsulosine selon la revendication 23-24, qui comprend du phosphate de calcium.

26. Utilisation de tamsulosine selon les revendications 1-25, qui comprend du lactose, de l'HPMC, un phosphate de calcium et du stéarate de magnésium.

27. Utilisation de tamsulosine selon les revendications 1-26, dans laquelle ledit comprimé est un comprimé à prendre une fois par jour.

28. Utilisation de tamsulosine selon les revendications 1-27, qui est un comprimé monolithique ne comprenant pas de délitant.

29. Utilisation de tamsulosine dans la fabrication d'un comprimé pharmaceutique monolithique, comprenant
- de 0,1 à 10 mg de tamsulosine qui consiste en un énantiomère (R) de tamsulosine ou d'un de ses sels pharmaceutiquement acceptables en dérivant,
- de 10% en poids à 90% en poids d'hydroxypropylméthylcellulose et
- un poids total du comprimé de 10 à 300 mg,
pour le traitement ou l'amélioration de l'hyperplasie bénigne de la prostate, le comprimé étant pris à jeun.

30. Utilisation de tamsulosine selon la revendication 29, dans laquelle ledit poids total du comprimé est compris entre 25 et 250 mg.

31. Utilisation de tamsulosine selon la revendication 30, dans laquelle ledit poids total du comprimé est compris entre 80 et 100 mg.

32. Utilisation de tamsulosine selon les revendications 29-31, qui comprend ladite hydroxypropylméthylcellulose en une quantité dans la gamme de 25% en poids à 40% en poids.

33. Utilisation de tamsulosine selon les revendications 29-32, qui comprend ladite hydroxypropylméthylcellulose en une quantité dans la gamme de 30% en poids à 40% en poids.

34. Utilisation de tamsulosine selon les revendications 29-33, dans laquelle ledit comprimé comprend ladite hydroxypropylméthylcellulose en une quantité dans la gamme de 30% en poids à 35% en poids.

35. Utilisation de tamsulosine selon les revendications 29-34, dans laquelle ledit comprimé comprend en outre un phosphate de calcium, du lactose, du mannitol ou une combinaison de ceux-ci.

36. Utilisation de tamsulosine selon la revendication 35, dans laquelle ledit comprimé comprend du phosphate de calcium dibasique anhydre.

37. Utilisation de tamsulosine selon la revendication 29-36, qui ne contient pas de revêtement entérique.
